# EUROPEAN PATENT SPECIFICATION

(11) **EP 1 934 251 B1**
(45) Date of publication and mention of the grant of the patent: **16.06.2010**
(21) Application number: 06831859.1
(22) Date of filing: 13.10.2006
(51) Int. Cl.: C07K 14/54, G01N 33/68

(54) **INTERLEUKIN-8 LABELLED VIA ONE OR MORE SITE SPECIFICALLY CONJUGATED HYDRAZINONICOTINAMIDE (HYNIC) MOIETIES AND ITS USE IN DIAGNOSIS OF INFECTION AND INFLAMMATION**
DURCH EIN ODER MEHRERE STANDORTSPEZIFISCH KONJUGIERTE HYDRAZIN-NIKOTIN-AMID(HYNIC)-TEILE MARKIERTES INTERLEUKIN-8 UND SEINE VERWENDUNG ZUR DIAGNOSTIZIERUNG VON INFEKTIONEN UND ENTZÜNDUNGEN
INTERLEUKINE 8 MARQUEE AU MOYEN D'UNE OU PLUSIEURS FRACTIONS HYDRAZINONICOTINAMIDE (HYNIC) CONJUGUEES DE MANIERE REGIOSPECIFIQUE ET SON UTILISATION DANS LE DIAGNOSTIC D'UNE INFECTION ET D'UNE INFLAMMATION

(30) Priority: 14.10.2005 EP 05077365
(43) Date of publication of application: 25.06.2008
(73) Proprietor: Stichting Katholieke Universiteit, 6500 HB Nijmegen (NL)
(72) Inventor: RENNEN, Huub, 6500 HB Nijmegen (NL); BOERMAN, Otto, 6500 HB Nijmegen (NL)
(74) Representative: Jones, Nicholas Andrew
(86) International application number: PCT/IB2006/003899
(87) International publication number: WO 2007/042946

(56) References cited:
- WO-A-02/082044
- WO-A-03/014158
- US-B1- 6 251 364
- RENNEN HJJM ET AL.: "Specific and Rapid Scintigraphic Detection of Infection with 99m-Tc-Labeled Interleukin-8" THE JOURNAL OF NUCLEAR MEDICINE, vol. 42, 2001, pages 117-123, XP002438960 cited in the application
- RENNEN HJJM ET AL.: "Effects of Coligand Variation on the In Vivo Characteristics of Tc-99m-Labeled Interleukin-8 in Detection of Infection" BIOCONJUGATE CHEMISTRY, vol. 13, 2002, pages 370-377, XP002438961 cited in the application
- RENNEN HJJM ET AL: "99m-Tc-Labeled Interleukin-8 for Scintigraphic Detection of Pulmonary infections" CHEST, vol. 126, no. 6, December 2004 (2004-12), pages 1954-1961, XP002438962 cited in the application
- BLEEKER-ROVERS CP ET AL.: "99m-Tc-Labeled Interleukin-8 for Scintigraphic Detection of Pulmonary infections: First Clinical Evaluation" THE JOURNAL OF NUCLEAR MEDICINE, vol. 48, no. 3, March 2007 (2007-03), pages 337-343, XP002438963
- GREENLAND WEP ET AL: "Solid-Phase Synthesis of Peptide Radiopharmaceuticals Using Fmoc-N.epsilon-(Hynic-Boc)-Lysine, a Technecium-Binding Amino Acid: Application to Tc-99m-Labeled Salmon Calcitonin" JOURNAL OF MEDICINAL CHEMISTRY, vol. 46, 27 March 2003 (2003-03-27), pages 1751-1757,

## Description

The present invention relates to compounds which are labelled via one or more hydrazinonicotinamide (HYNIC) moieties selectively conjugated to an N-terminal amino acid and/or to one or more internal lysines of Interleukin-8 (IL-8) or an analog or derivative thereof.

Scintigraphic detection of the localization of acute, sub-acute and chronic infection and inflammation is a challenging problem in clinical practice, because it may have important implications for the management of patients with infectious or inflammatory diseases. In order to enable clinicians to rapidly initiate the most appropriate treatment, adequate delineation of inflammatory foci in these patients is of clinical importance. If the clinical history and physical examination are indecisive, the clinician can choose from several diagnostic modalities to determine the localization, the extent and the severity of the disease. New highly sensitive radiological investigations like magnetic resonance imaging and spiral computerized tomography are able to locate relatively small focal abnormalities. However, these radiological methods rely upon morphological changes. They are therefore less accurate in the early stages of infection and are unable to discriminate active processes from anatomical changes due to a cured infection or after surgery (scar tissue).

In nuclear medicine, a radiolabelled compound - the radiopharmaceutical - is injected and the distribution of the radioactivity throughout the body is visualized using a dedicated gamma camera. Scintigraphic images do not depend upon morphological changes, but are based upon physiochemical processes in tissues. Therefore, scintigraphic techniques can also visualize infectious foci in the early phases, when morphological changes are not yet apparent. In addition, scintigraphic imaging is an excellent non-invasive method of whole-body scanning that can determine the extent of the infectious or inflammatory disease throughout the body.

Human IL-8 is a small chemotactic protein, binding with high affinity to receptors on neutrophils. There are two naturally existing isoforms of human IL-8. The most abundant form is the 72 amino acid variant produced by monocytes. A 77 amino acid variant of IL-8 is produced by endothelial cells and is extended at the N-terminus by five amino acids. The shorter form is more potent that the longer form in attracting and activating neutrophils, but both forms are active at nanomolar concentrations. Previously, it was only the 72 amino acid isoform of IL-8 which had been used in preclinical and clinical studies in infection and inflammation imaging. The potential of radiolabelled IL-8 to image inflammation was reported for the first time by Hay et al [Nucl. Med. Commun., 1997, 18; 367-378]. The uptake of IL-8, radioiodinated via the chloramine-T method, in carrageenan-induced sterile inflammations in rats, peaked at 1-3 hours after injection and declined thereafter. In a pilot study in eight diabetic patients these investigators showed that ¹²³I-IL-8 could visualize active foot infections [Gross et al, J. Nucl. Med. Chem., 2001; 42; 1656-1659].

The labelling method appeared to have major effects on the *in vivo* biodistribution of the radioiodinated IL-8. The scintigraphic imaging characteristics of IL-8 labelled via the Bolton-Hunter method were clearly superior to IL-8 labelled via the iodogen method, despite similar *in vitro* cell binding characteristics [van der Laken et al, J. Nucl. Med. Chem., 2000; 41; 463-469]. The specific activity of this IL-8 preparation was relatively low; the imaging dose of ¹²³I-IL-8 (25 µg/kg) caused a transient drop in peripheral leukocyte counts to 45%, followed by a leukocytosis (170% of preinjection level) over several hours.

Recently, the development of a ^{99m}Tc-labelled IL-8 preparation using HYNIC as a chelator was described [Rennen et al, J. Nucl. Med., 2001; 42; 117-123; Rennen et al, Bioconjug. Chem., 2002; 13; 370-377; Rennen et al, J. Nucl. Med , 2003; 44; 1502-1509; Gratz et al, J. Nucl. Med., 2001; 42; 917-923; Gratz et al, J. Nucl. Med., 2001; 42; 917-923; Gratz et al, J. Nucl. Med., 2001; 42; 1257-1264; and Rennen et al, Chest. 2004 Dec, 126(6), 1954-1961]. This preparation showed excellent characteristics for imaging infection in four different models of infection and inflammation in rabbits. In rabbits with intramuscular infection induced by *E.coli,* uptake of ^{99m}Tc-labelled IL-8 in the abscess was rapid and high. Moreover, ^{99m}Tc-labelled IL-8 showed a remarkably fast clearance from non-target tissues. Abscess-to-muscle ratios exceeded 200 at 6 hours after injection.

In rabbits with chemically induced acute colitis, inflammatory lesions were scintigraphically visualized after injection of either IL-8 or purified granulocytes, both labelled with ^{99m}Tc. Within a few hours after injection, ^{99m}Tc-labelled IL-8 allowed an adequate evaluation of the inflamed colon. Absolute uptake in the inflamed foci in the colon was much higher for ^{99m}Tc-labelled IL-8 than for ^{99m}Tc-labelled granulocytes.

In a third study, the performance of ^{99m}Tc-labelled IL-8 was evaluated in an experimental model of acute osteomyelitis in rabbits. The results were compared with those obtained using the conventional and well-established agent ¹¹¹In-granulocytes, ⁶⁷Ga-citrate and ^{99m}Tc-MDP (MDP - methylene diphosphonate). In this rabbit model of osteomyelitis, ^{99m}Tc-IL-8 clearly delineated the osteomyelitis lesions. Although absolute uptake in the osteomyelitic area was lower than that obtained with ⁶⁷Ga-citrate and ^{99m}Tc-MDP, the target-to-background ratios significantly higher for ^{99m}Tc-IL-8.

Finally, ^{99m}Tc-labelled IL-8 was tested for its potential to image pulmonary infection in three experimental rabbit models: aspergillosis in immunocompromised rabbits, pneumococcal (gram-positive) pneumonia and E.coli-induced (gram-negative) pneumonia in immunocompetent rabbits. ^{99m}Tc-IL-8 enabled early (within 2h of injection) and excellent visualisation of localisation and extent of pulmonary infection in each of the three experimental models of pulmonary infection. Uptake of ^{99m}Tc-IL-8 in the foci of infection was generally high, and in the *E.coli* model, uptake in the foci of infection even exceeded uptake in the kidneys, the main clearing organs.

IL-8 is a proinflammatory chemotactic cytokine. For scintigraphic purposes, protein doses to be administered should be below levels that generate side effects. It was shown that a preparation with a high specific activity (80 MBq/µg) and high *in vitro* stability could be prepared when nicotinic acid was used as a co-ligand. Protein doses to be administered were as low as 70 ng/kg of bodyweight. At such low protein doses side effects are not to be expected in the human system. Initial results with ^{99m}Tc-IL-8 in patients with infectious and inflammatory disorders proved that ^{99m}Tc-IL-8 could indeed be safely administered at these dosage levels.

^{99m}Tc-IL-8 has favourable characteristics as an infection imaging agent: rapid accumulation in target tissue and rapid clearance from blood and non-target tissues. The activity is mainly cleared via the kidneys, which is an advantage over hepatobiliary clearance. High activity in the liver and bowel would have made this agent less suitable for imaging of the abdominal region.

^{99m}Tc-IL-8 offers several advantages over the two most commonly used radiopharmaceuticals for infection imaging, ⁶⁷Ga-citrate and radiolabelled leukocytes. The radionuclide ^{99m}Tc is preferred over ⁶⁷Ga because of its virtually ideal physical characteristics (short half-life, ideal energy, and low radiation burden), its cost-effectiveness and general availability. ⁶⁷Ga has a longer physical half-life and high energy gamma radiations, causing high radiation absorbed doses and generating images of lower resolution. Generally, ⁶⁷Ga-citrate shows relatively slow pharmacokinetics. As a consequence, a long interval between injection of the radiopharmaceutical and imaging is required. Typically, ⁶⁷Ga-imaging is performed between 48 and 72 hours post-injection. In imaging rabbits using ^{99m}Tc-IL-8, a 2 hour interval between injection and imaging was sufficient.

In comparison with radiolabelled leukocytes, the preparation of ^{99m}Tc-IL-8 is easy and rapid, ready within 30 minutes and with no need for further purification. The preparation of the radiolabelled leukocytes, be it either with ¹¹¹In or ^{99m}Tc, is cumbersome, time-consuming, and not possible in granulocytopenic patients. The procedure of taking blood from a patient, purification of the leukocytes and labelling of these cells takes a trained technician approximately 3 hours. Cells should be handled cautiously in order to preserve their capacity to migrate to the inflamed area upon re-injection. In addition, the need to handle potentially contaminated blood could lead to transmission of blood-born pathogens such as HIV and presents serious risks to both personnel and patient. The convenient method of labelling leukocytes *in vivo* with ^{99m}Tc-IL-8 reflects the traditional method of labelling *ex vivo,* with all the advantages of the new method detailed above.

In the studies carried out and published until now using HYNIC as a ^{99m}Tc chelating group, the HYNIC group(s) was (were) randomly linked to primary amino groups of the IL-8 molecule. IL-8 contains one α-amino group (N-terminus) and nine ε-amino groups (Lysine sidechains). The receptor binding affinity of the IL-8 might be reduced if active sites of the peptide are modified chemically. The inventors prepared a series of HYNIC-IL-8 conjugates by using different molar conjugation ratios (IL-8:S-HYNIC of 1:1 to 1:10) and different reaction times (3-60 minutes). It was shown that the leukocyte receptor binding capacity of ^{99m}Tc-labelled HYNIC-conjugated IL-8 was greatly reduced when more HYNIC moieties were conjugated to IL-8. This indicates that some of the ε-amino groups of the IL-8 molecule cannot be modified in this way without negatively impacting upon receptor binding.

Therefore, in accordance with the present invention, there is provided a HYNIC-IL-8 conjugate product comprising greater than 80% quantity of one specific HYNIC-IL-8 conjugate selected from an interleukin-8, or human native IL-8₁₋₇₂ having modifications, deletions or exchanges in residues 1-3 and 67-72, labelled via one or more hydrazinonicotinamide (HYNIC) moieties site-specifically conjugated to one or more internal lysines and/or an N-terminal amino acid.

According to the invention, the HYNIC moieties may be conjugated to the N-terminal amino acid and site specifically to one or more internal lysines. Alternatively, the HYNIC moieties may be conjugated solely to the N-terminal amino acid or to one or more internal lysines. Preferably, they are conjugated at least to the N-terminal amino acid and to any of 0, 1, 2, 3, 4, 5, 6, 7, 8 or 9 internal lysines.

The invention allows the synthesis of specific well-defined HYNIC IL-8 conjugates, rather than the existing random conjugations. The synthesis of HYNIC-IL-8 conjugates wherein the well-defined HYNIC-IL-8 conjugates comprise a greater than about 80% quantity of the overall product is enabled. Selectivity is preferably greater than about 85%, more preferably greater than about 90%, still more preferably greater than about 95%. Most preferably, the HYNIC-IL-8 conjugates comprise a greater than 98% quantity of a well-defined HYNIC conjugated IL-8 product.

The IL-8 is preferably labelled by a radiolabel. More preferably, the radiolabel is selected from ^{99m}Tc and ^{94m}Tc. Most preferably, the radiolabel is ^{99m}Tc.

Both the 72 and 77 amino acid isoforms can be modified according to the present invention, although it is the 72 amino acid isoform which is preferred.

By analogs and derivatives of IL-8, it is meant IL-8 having modifications, deletions or exchanges in residues 1-3 and 67-72 of human native IL-8₁₋₇₂. It is the IL-8₄₋₆₆ core which is essential for the biological activity of IL-8. The residues 1-3 and 67-72 can therefore be deleted, modified or exchanged without having a significant effect upon the biological functions of IL-8.

Also provided in accordance with the invention is a method of site-specifically conjugating one or more HYNIC moieties to Interleukin-8, comprising:
a) providing a protected immobilised IL-8;
b) coupling hydrazinonicotinic acid to the immobilised IL-8;
c) deprotecting the HYNIC-conjugated IL-8;
d) cleavage of deprotected HYNIC-conjugated IL-8 from a solid support.

The method can also be used in the stepwise site-specific conjugation of more than one HYNIC moieties to the IL-8. This can be achieved by providing in step (a) a protected immobilised IL-8 which has already been site-specifically conjugated by one or more HYNIC moieties.

Preferably, one HYNIC moiety is conjugated to the N-terminal amino acid using this method. The method is preferably carried out using solid phase peptide synthesis.

The method of the invention is capable of achieving a selectivity of HYNIC conjugation such that the resultant product has a greater than about 80% quantity of a desired HYNIC-conjugated IL-8, preferably greater than about 85%, more preferably greater than about 90%, still more preferably greater than about 95%, and most preferably greater than about 98%.

The method is most preferably used for the conjugation of one HYNIC moiety solely to the N-terminal amino acid.

Preferably, the method further comprises contacting the product from step (d) with a labelling source. The labelling source preferably comprises a radiolabel, such as ^{99m}Tc. A preferred source of ^{99m}Tc is ^{99m}TcO₄.

The peptide IL-8 is synthesized on an automated peptide synthesizer. It could be immobilized on any suitable support. After coupling of hydrazinonicotinic acid (it is essential to protect the hydrazine group) to IL-8, the anchored HYNIC conjugated peptide is deprotected and cleaved form the solid support.

Activation of the IL-8 is preferably carried out in the presence of one or more coupling agents. While other coupling agents can be used, the coupling agents that are preferably used are O-benzotriazole-N,N,N',N'-tetramethyl-uronium-hexafluorophosphate (HBTU), 1-hydroxybenzotriazole hydrate (HOBt), 2-(1H-7-azabenzotriazol-1-yl)-1,1,3,3-tetramethyl uronium hexafluorophosphate (HATU), or 1-hydroxy-7-azabenzotriazole (HOAt).

Deprotection of the IL-8 can be carried out using any suitable secondary amine, such as piperidine, followed by washing with e.g. N-methyl-2-pyrrolidine (NMP) to remove the amine.

Cleavage of the deprotected anchored HYNIC conjugated IL-8 from the solid support can be done by using e.g. a mixture of trifluoroacetic acid, water, 1,2-ethanedithiol and triisopropylsilane. The IL-8 is then purified.

The IL-8 provided in step (a) may be immobilised on any suitable solid support, such as a resin.

The IL-8 is preferably protected using Fmoc or Boc as protecting groups. Conjugation of HYNIC to the IL-8 during solid phase synthesis is essential.

The invention also includes a use of the IL-8 in the preparation of a pharmaceutical composition for diagnosis of infection and inflammation.

The human IL-8 analogue labelled with e.g. ^{99m}Tc via a HYNIC-moiety/moieties which is conjugated to the N-terminal amino acid and/or one or more internal lysines may provide one or more of the following distinct advantages:
- The ^{99m}Tc-chelating HYNIC group is attached to the IL-8 in a well-defined, site-specific manner;
- The introduced HYNIC group is located at such a position that it does not interfere with receptor interaction, thus preserving the high receptor binding affinity of the IL-8; and
- The HYNIC-IL-8 conjugate contains a predetermined quantity of HYNIC moieties, such as exactly one when only the N-terminal is labelled, which will allow the preparation of ^{99m}Te-IL-8 preparations of higher specific activity (MBq/nmol), which will allow administration of lower peptide doses to patients, thus reducing the occurrence of undesirable side effects.

The HYNIC-IL-8 compounds of the invention are capable of achieving a receptor binding capacity of >70%, preferably >80%, more preferably >85%, still more preferably >90%, and most preferably >95%.

The invention is also described with reference to the accompanying Figures:

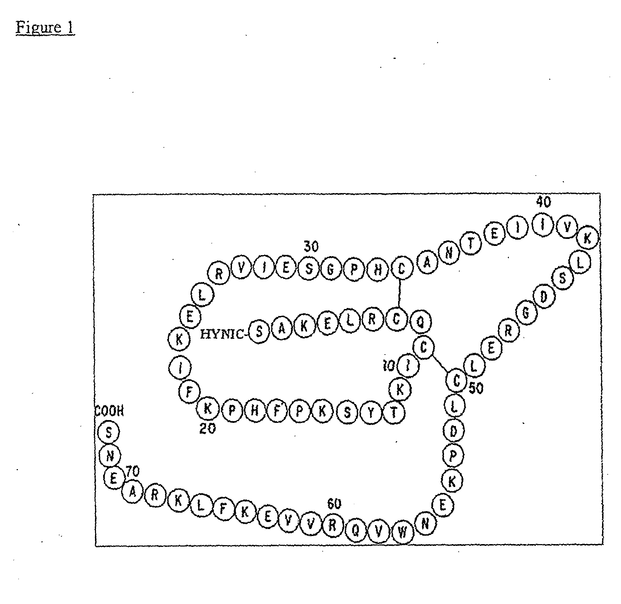

Figure 1 shows a structure of the CYNIC N-terminally conjugated to IL-8.
Figure 2 shows scintigraphic images of a representative rabbit (one out of a group of 5 rabbits) with intramuscular infection at 1h, 3h, and 5h post-injection.

The invention is further described with reference to the following Examples, which are intended to be purely illustrative, and in no way limiting upon the scope of the invention.

### EXAMPLES

### 1. Synthesis of N-terminally conjugated HYNIC₁-IL-8:

The amino acid sequence of human 72 amino acid IL-8 is:

1	11	21	31	41
SAKELRCQCI	KTYSKPFHPK	FIKELRVIES	GPHCANTEII	VKLSDGRELC
51	61	71
LDPKENWVQR	VVEKFLKRAE	NS

The HYNIC group is introduced into IL-8 at the N-terminal serine residue (Ser¹) of the molecule which can be chemically modified without affecting the receptor binding capacity of the peptide.

### Synthesis of peptide IL-8:

The immobilized peptide was assembled on an automatic ABI 433A Peptide Synthesizer using the ABI FastMoc 0.25 mmol protocols (References 1, 2), except that the coupling time was 45 min instead of 20 min. The synthesis was carried out on ArgoGel-Wang resin (Argonaut Technologies, capacity 0.37 mmol/g).

After cleavage of the Fmoc-group by means of a 20% piperidine solution in N-methyl-2-pyrrolidone (=NMP) (3 min and 7.6 min), the resin was washed with NMP (5 x). Subsequently, 4 equivalents (1 mmol) of the appropriate amino acid were dissolved in NMP (2 mL), and HBTU/HOBt (1 mmol, 2.78 mL of 0.36 M) in NMP was added. To this mixture N,N-diisopropylethylamine (=DiPEA) (1 mL, 2 M) in NMP was added, and the activated amino acid was then transferred to the reaction vessel. After 45 min, the reaction vessel was drained and the resin was washed with NMP (3 x). The deprotection and coupling reactions were followed by monitoring the dibenzofulvene-piperidine adduct at 301 nm. The last amino acid coupling cycle was followed by removal of the Fmoc-group and coupling of Fmoc-6-hydrazinonicotinic acid (4 eq., HBTU/HOBt (4 eq.), DiPEA(8 eq.) in NMP for 45 minutes) to the free N-terminus of the protected peptide attached to resin. The coupling of Fmoc-6-hydrazinonicotinic acid was repeated once to ensure completion of the reaction. This last coupling cycle was followed by removal of the Fmoc-group by a piperidine solution, washing the resin with NMP (5 x) and dichloromethane (DCM) (6 x). Finally the resin was removed from the reaction vessel; washed with DCM, MeOH and ether, and dried *in vacuo* over P₂O₅.

The anchored peptide thus obtained was deprotected and cleaved from the solid support by treatment with 45 mL trifluoroacetic acid (=TFA), 2.5 mL water, 1.25 mL 1,2-ethanedithiol (=EDT) and 1.25 mL triisopropylsilane (=TIS), for 3 h at room temperature. The mixture was then filtered and the residue washed thoroughly with TFA (3 x 5 mL). The reaction mixture was concentrated *in vacuo* to a volume of approximately 10 mL and the residue was added dropwise to 90 mL MTBE/n-hexane (1/1, v/v) solution. The precipitate was collected by centrifugation (3000 rpm, 10 min), the supernatant was decanted, and the pellet was resuspended in MTBE (100 mL) and centrifuged again. This was repeated twice. After this, the pellet was dissolved in acetonitrile/water (1/1, v/v) (ca. 60 mL) and lyophilized to obtain the crude peptide as a white fluffy solid.

In batches of 50 mg the crude peptide was dissolved and treated with 4 mL of 6 M guanidine HCl, 0.1 M Tris HCl, 20% mercaptoethanol at pH 8.5 and stirred at 45 °C for 2 h and then acidified with 1 mL acetic acid.

This mixture containing the reduced crude peptide was loaded onto a preparative Phenomenex column (250 x 21.2 mm, C18, 110 A, 10 µm) and the retained material eluted with a 0-60% water-acetonitrile gradient in 0.1 % TFA over 240 min at a flow rate of 12.5 mL/min using an automatic Shimadzu Prep LCMS system Fractions containing the correct mass peak were re-run for purity assessment on a Phenomenex (250 x 4.6 mm, C18, 110Å, 5 µm) column and the ones containing the major peak were pooled and lyophilized. To fold and oxidise the peptide the material was reconstituted in phosphate buffered saline overnight.

### References:

*1.* Applied Biosystems Model 433A Peptide Synthesizer User's Manual, June 1993, Version 1.0.
*2.* Applied Biosystems Research News, June 1993, Model 433A Peptide Synthesizer, 1-12.

### Abbreviations used:

HBTU=2-(1H-benzotriazole-1-yl)-1,1,3,3-tetramethyluronium hexafluorophosphate
HOBt = 1-hydroxybenzotriazole
MTBE = *tert*-butyl methyl ether

### Labeling of N-terminally conjugated HYNIC₁-IL-8 with Technetium-99m

Lyophilized co-ligand kits were prepared containing 30 µg SnSO₄, 45 mg tricine and 4 mg nicotinic acid. 600 µl of a 0.9 % NaCl solution was added to a co-ligand kit immediately before the start of the radiolabelling.

To a 10 µg HYNIC₁-IL-8 sample was added 400 µl of the reconstituted co-ligand kit together with 400 MBq ^{99m}TcO₄ in saline. The mixture was incubated at 70 °C for 30 minutes. The radiochemical purity of the preparation was determined by instant thin-layer chromatography (ITLC) on ITLC-SG strips (Gelman Laboratories, Ann Arbor, MI) with 0.1 M citrate, pH 6.0, as the mobile phase.

Following the labeling reactions the reaction mixtures were diluted with Phosphate Buffered Saline (PBS), 0.5% Bovine Serum Albumin (BSA) to a final concentration of 40 MBq/ml, ready for i.v. administration in the rabbits.

### 2. Synthesis of IL-8 having HYNIC conjugated to one or more internal lysines:

The HYNIC group is conjugated to one or more of the nine internal lysines (abbreviated by "K" in the amino acid sequence of IL-8). Here, as amino acid building block in solid phase peptide synthesis, Fmoc-Lys(HYNIC-BOC)-OH is used instead of Fmoc-Lys(BOC)-OH for introduction of a HYNIC group at a particular position "x" in HYNICₓ-IL-8.

In addition, IL-8 derivatives can be synthesized by solid phase peptide synthesis, with HYNIC conjugated to the N-terminus (as in Example 1) as well as to one or more internal lysines (as in Example 2).

### Animal studies

Animal studies were carried out in accordance with the guidelines of the local animal welfare committee. Abscesses were induced in the left thigh muscle of ten female New Zealand white rabbits (2.4-2.7 kg) with 10¹¹ colony forming units (cfu) of *Escherichia coli* in 0.5 ml. During the procedure, the rabbits were sedated with a subcutaneous injection of a 0.6 ml mixture of fentanyl 0.315 mg/ml and fluanisone 10 mg/ml (Hypnorm, Janssens Pharmaceutical, Buckinghamshire, UK).

After 24 hours, when swelling of the muscle was apparent, five rabbits were injected with 40 MBq ^{99m}Tc-HYNIC₁-IL-8 via the ear vein. Three of the rabbits were used for gamma camera imaging. For imaging, rabbits were immobilized, placed prone on the gamma camera and injected with either ^{99m}Tc-HYNIC₁-IL-8 or ^{99m}Tc-HYNICₓ-IL-8 (x = 2-10) in the lateral ear vein. Images were recorded at 1 min, 1, 3 and 5 h post-injection (p.i.) with a single-head gamma camera (Orbiter, Siemens Medical Systems Inc., Hoffman Estates, IL) equipped with a parallel-hole low-energy all purpose collimator. Images (200,000-300,000 counts per image) were obtained and digitally stored in a 256 x 256 matrix.

After completion of the final imaging and blood sampling (5 h p.i.), the rabbits were euthanized with a lethal dose of sodium phenobarbital. Samples of blood, infected thigh muscle, uninfected contralateral thigh muscle, lung, spleen, liver, kidneys and intestines were collected. The dissected tissues were weighed and counted in a gamma counter. Injection standards were counted simultaneously to correct for radioactive decay. The measured activity in samples was expressed as percentage of injected dose per gram tissue (%ID/g). Abscess-to-contralateral muscle ratios and abscess-to-blood ratios were calculated.

### RESULTS

### ^{99m}Tc-labeling of HYNIC₁-IL-8

The radiochemical purity of ^{99m}Tc-HYNIC₁-IL-8 was 98%, as determined by instant thin-layer chromatography. The specific activities of the preparation used in this study was 40 MBq per microgram IL-8.

### Animal studies

The biodistribution of ^{99m}Te-HYNIC₁-IL-8 in rabbits with intramuscular infection is summarized in the table below.

| | ***Tc-99m-HYNIC1-IL8*** | | |
|---|---|---|---|
| | MEAN | SD | s.e.m. |
| | %ID/g | | |
| blood | **0,009** | 0,001 | 0,001 |
| muscle | **0,0023** | 0,0014 | 0,0006 |
| abscess | **0,090** | 0,02 | 0,01 |
| lung | **0,22** | 0,08 | 0,04 |
| spleen | **0,31** | 0,07 | 0,03 |
| kidney | **0,90** | 0,19 | 0,09 |
| liver | **0,099** | 0,013 | 0,006 |
| intestine | **0,020** | 0,002 | 0,001 |
| | | | |
| | ratios | | |
| abscess/muscle | **49** | 25 | 11 |
| abscess/blood | **10** | 2 | 1 |

| | | | |
|---|---|---|---|
| (SD = Standard Deviation; s.e.m. = Standard Error of the Mean) | | | |

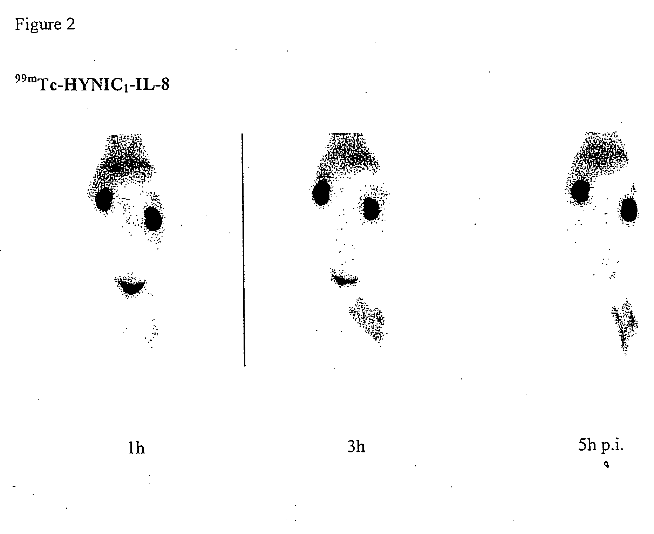

Scintigraphic images of a representative rabbit (one out of the group of 5 rabbits) with intramuscular infection at 1 h, 3 h and 5 h p.i. are shown in Figure 2.

## Claims

1. A HYNIC-IL-8 conjugate product comprising greater than 80% quantity of one specific HYNIC-IL-8 conjugate selected from an interleukin-8, or human native IL-8₁₋₇₂ having modifications, deletions or exchanges in residues 1-3 and 67-72, labelled via one or more hydrazinonicotinamide (HYNIC) moieties site-specifically conjugated to one or more internal lysines and/or an N-terminal amino acid.

2. HYNIC-IL-8 conjugate product according to claim 1, comprising HYNIC moieties site-specifically conjugated to the N-terminal amino acid and one or more internal lysines.

3. HYNIC-IL-8 conjugate product according to claim 2, comprising HYNIC moieties site-specifically conjugated to the N-terminal amino acid and one internal lysine.

4. HYNIC-IL-8 conjugate product according to claim 1, comprising a HYNIC moiety conjugated solely to the N-terminal amino acid.

5. HYNIC-IL-8 conjugate product according to claim 1, comprising a HYNIC moiety site-specifically conjugated to one or more internal lysines.

6. HYNIC-IL-8 conjugate product according to any preceding claim, wherein the Interleukin-8 is labelled by a radiolabel.

7. HYNIC-IL-8 conjugate product according to claim 6, wherein the radiolabel is ^{99m}Tc.

8. HYNIC-IL-8 conjugate product according to any preceding claim, comprising a 72 amino acid isoform of the Interleukin-8.

9. A method of site-specifically conjugating one or more HYNIC moieties to Interleukin-8, comprising:
a) providing a protected immobilised Interleukin-8;
b) coupling hydrazinonicotinic acid to immobilised Interleukin-8;
c) deprotecting the HYNIC-conjugated Interleukin-8;
d) cleavage of deprotected HYNIC-conjugated Interleukin-8 from a solid support.

10. A method according to claim 9, wherein the protected immobilised Interleukin-8 provided in step (a) has previously been site-specifically conjugated by one or more HYNIC moieties.

11. A method according to claim 9 or claim 10, which results in a HYNIC-conjugated Interleukin-8 product comprising a greater than about 80% quantity of one specific well-defmed HYNIC-conjugated Interleukin-8.

12. A method according to any of claims 9-11, wherein one HYNIC moiety is conjugated solely to the N-terminal amino acid of the Interleukin-8.

13. A method according to any of claims 9-12, wherein the method is carried out using solid phase peptide synthesis.

14. A method according to any of claims 9-13, further comprising contacting the product from step (d) with a labelling source.

15. A method according to claim 14, wherein the labelling source comprises a radiolabel.

16. A method according to claim 15, wherein the radiolabel is ^{99m}Tc.

17. Use of the HYNIC-IL-8 conjugate product as claimed in any of claims 1-8 in the preparation of a pharmaceutical composition for diagnosis of infection and inflammation.

18. The HYNIC-IL-8 conjugate product as claimed in any of claims 1-8 for use in diagnosis of infection and inflammation.

## Patentansprüche

1. HYNIC-IL-8-Konjugatprodukt, umfassend eine Menge von mehr als 80 % eines spezifischen HYNIC-IL-8-Konjugats, ausgewählt aus einem Interleukin-8, oder menschlichem nativem IL-8₁₋₇₂ mit Modifikationen, Deletionen oder Austausch an den Resten 1-3 und 67-72, markiert über eine oder mehrere Hydrazinonicotinamidgruppen (HYNIC), die ortsspezifisch an eines oder mehrere interne Lysine und/oder N-terminale Aminosäuren konjugiert sind.

2. HYNIC-IL-8-Konjugatprodukt nach Anspruch 1, umfassend an die N-terminale Aminosäure und eines oder mehrere interne Lysine ortsspezifisch konjugierte HYNIC-Gruppen.

3. HYNIC-IL-8-Konjugatprodukt nach Anspruch 2, umfassend an die N-terminale Aminosäure und eines oder mehrere interne Lysine ortsspezifisch konjugierte HYNIC-Gruppen.

4. HYNIC-IL-8-Konjugatprodukt nach Anspruch 1, umfassend eine nur an die N-terminale Aminosäure konjugierte HYNIC-Gruppe.

5. HYNIC-IL-8-Konjugatprodukt nach Anspruch 1, umfassend eine ortsspezifisch an eines oder mehrere interne Lysine konjugierte HYNIC-Gruppe.

6. HYNIC-IL-8-Konjugatprodukt nach einem der vorangehenden Ansprüche, wobei das Interleukin-8 mit einem radioaktiven Marker markiert ist.

7. HYNIC-IL-8-Konjugatprodukt nach Anspruch 6, wobei der radioaktive Marker ^{99m}Tc ist.

8. HYNIC-IL-8-Konjugatprodukt nach einem der vorangehenden Ansprüche, umfassend eine Aminosäure-72-Isoform des Interleukins-8.

9. Verfahren zur ortsspezifischen Konjugation einer oder mehrerer HYNIC-Gruppen an Interleukin-8, umfassend
a) Bereitstellen eines geschützten immobilisierten Interleukins-8,
b) Kuppeln von Hydrazinonicotinsäure an das immobilisierte Interleukin-8,
c) Abspalten der Schutzgruppe vom HYNIC-konjugierten Interleukin-8,
d) Spalten des freien HYNIC-konjuguierten Interleukins-8 von einem festen Träger.

10. Verfahren nach Anspruch 9, wobei das im Schritt a) bereitgestellte geschützte immobilisierte Interleukin-8 vorher mit einer oder mehreren HYNIC-Gruppen ortsspezifisch konjugiert wurde.

11. Verfahren nach Anspruch 9 oder Anspruch 10, das zu einem HYNIC-konjugierten Interlekin-8-Produkt führt, welches eine Menge von mehr als etwa 80 Prozent eines spezifischen, wohldefinierten HYNIC-konjugierten Interleukins-8 umfasst.

12. Verfahren nach einem der Ansprüche 9-11, wobei eine HYNIC-Gruppe nur an die N-terminale Aminosäure des Interleukins-8 konjugiert ist.

13. Verfahren nach einem der Ansprüche 9-12, wobei das Verfahren unter Anwendung der Festphasenpeptidsynthese ausgeführt wird.

14. Verfahren nach einem der Ansprüche 9-13, ferner das Inkontaktbringen des Produkts aus Schritt d) mit einer markierenden Quelle umfassend.

15. Verfahren nach Anspruch 14, wobei die markierende Quelle einen radioaktiven Marker umfasst.

16. Verfahren nach Anspruch 15, wobei der radioaktive Marker ^{99m}Tc ist.

17. Verwendung des HYNIC-IL-8-Konjugatprodukts nach einem der Ansprüche 1-8 bei der Herstellung einer pharmazeutischen Zusammensetzung zur Diagnose von Infektion und Entzündung.

18. HYNIC-IL-8-Konjugatprodukt nach einem der Ansprüche 1-8 zur Verwendung bei der Diagnose von Infektion und Entzündung.

## Revendications

1. Composé conjugué HYNIC-IL-8 renfermant une quantité supérieure à 80 % d'un conjugué HYNIC-IL-8 spécifique choisi parmi une interleukine-8 ou une IL-9₁₋₇₂ native humaine présentant des modifications, des suppressions ou des échanges dans les restes 1-3 et 67-72, marquée au moyen d'au moins une fraction d'hydrazinonicotinamide (HYNIC) conjuguée de manière régiospécifique à au moins une lysine interne et/ou un acide aminé N-terminal.

2. Composé conjugué HYNIC-IL-8 selon la revendication 1, renfermant des fractions d'HYNIC conjuguées de manière régiospécifique à l'acide aminé N-terminal et à au moins une lysine interne.

3. Composé conjugué HYNIC-IL-8 selon la revendication 2, renfermant des fractions d'HYNIC conjuguées de manière régiospécifique à l'acide animé N-terminal et à une lysine interne.

4. Composé conjugué HYNIC-IL-8 selon la revendication 1, renfermant une fraction d'HYNIC conjuguée uniquement à l'acide aminé N-terminal.

5. Composé conjugué HYNIC-IL-8 selon la revendication 1, renfermant une fraction d'HYNIC de manière régiospécifique à au moins une lysine interne.

6. Composé conjugué HYNIC-IL-8 selon l'une quelconque des revendications 1 à 5, dans lequel l'interleukine-8 est marquée par un radio marqueur.

7. Composé conjugué HYNIC-IL-8 conjugué selon la revendication 6, dans lequel le radio marqueur est ^{99m}Tc.

8. Composé conjugué HYNIC-IL-8 selon l'une des revendications 1 à 7, comprenant une protéine iso-forme à 72 acides aminés de l'interleukine-8.

9. Procédé permettant de conjuguer de manière régiospécifique au moins une fraction d'HYNIC à l'interleukine-8 comprenant les étapes consistant à :
a) Se procurer une interleukine-8 immobilisée protégée,
b) Accoupler l'acide hydrazinonicotinique à interleukine-8 immobilisée,
c) Supprimer la protection de l'interleukine-8 conjuguée à HYNIC,
d) Cliver l'interleukine-8 conjuguée à HYNIC non protégée d'un support solide.

10. Procédé conforme à la revendication 9, selon lequel l'interleukine-8 immobilisée protégée mise en oeuvre à l'étape (a) a préalablement été conjuguée de manière régiospécifique à au moins une fraction d'HYNIC.

11. Procédé conforme à la revendication 9, ou à la revendication 10 permettant d'obtenir un composé d'interleukine-8 conjugué à HYNIC renfermant une quantité spécifique environ supérieure à 80 % d'une interleukine-8 conjuguée à HYNIC bien définie.

12. Procédé conforme à l'une des revendications 9 à 11, selon lequel une fraction d'HYNIC est uniquement conjuguée à l'acide aminé N-terminal de l'interleukine-8.

13. Procédé conforme à l'une des revendications 9 à 12, selon lequel le procédé est mis en oeuvre en utilisant la synthèse peptidique en phase solide.

14. Procédé conforme à l'une des revendications 9 à 13, comprenant en outre une étape consistant à mettre le composé obtenu à l'étape (d) en contact avec une source de marquage.

15. Procédé conforme à la revendication 14, selon lequel la source de marquage renferme un radio marqueur.

16. Procédé conforme à la revendication 15 selon lequel le radio marqueur est ^{99m}Tc.

17. Utilisation du composé conjugué HYNIC-IL-8 selon l'une des revendications 1 à 8 pour la préparation d'une composition pharmaceutique pour le diagnostic d'une infection et d'une inflammation.

18. Composé conjugué HYNIC-IL-8 selon l'une des revendications 1 à 8 destiné à être utilisé pour le diagnostic d'une infection et d'une inflammation.
